# EUROPÄISCHE PATENTANMELDUNG

(11) **EP 0 905 637 A1**
(43) Veröffentlichungstag der Anmeldung: **31.03.1999**
(21) Anmeldenummer: 98117387.5
(22) Anmeldetag: 14.09.1998
(51) Int. Cl.: G06F 19/00

(54) **Medizinische Systemarchitektur**

(30) Priorität: 25.09.1997 DE 19742234
(71) Anmelder: SIEMENS AKTIENGESELLSCHAFT, 80333 München (DE)
(72) Erfinder: Striebel, Werner, 91207 Lauf (DE); Prihoda, Heinz, 90411 Nürnberg (DE)

(57) **Zusammenfassung**

Die Erfindung betrifft eine medizinische Systemarchitektur mit einer Vorrichtung (1 bis 4, 26) zur Erfassung von medizinischen Bilder und/oder Daten, mit einer über ein Datennetz (17) zur Übertragung der erfassten Bilder und/oder Daten mit der Vorrichtung (1 bis 4, 26) verbundene, externe Speichervorrichtung (25) zur Abspeicherung der erfassten Bilder und/oder Daten, mit einer über ein Datennetz (17) mit der Speichervorrichtung (25) verbundenen Befundungsstation (27) zur Auswertung der erfassten Bilder und/oder Daten, wobei die Daten der Auswertung in der Speichervorrichtung (25) abgespeichert werden, so daß sie von einer Ausgabestation (28) über ein Datennetz (17) abrufbar sind.

## Beschreibung

Die Erfindung betrifft eine medizinische Systemarchitektur mit einer Vorrichtung zur Erfassung von medizinischen Bilder und/oder Daten, mit einer Speichervorrichtung zur Abspeicherung der erfassten Bilder und/oder Daten, mit einer Befundungsstation zur Auswertung der erfassten Bilder und/oder Daten.

Aus dem Buch "Bildgebende Systeme für die medizinische Diagnostik", herausgegeben von H. Morneburg, 3. Auflage, 1995, Seiten 684ff sind medizinische Systemarchitekturen bekannt, bei denen zum Abruf von Patientendaten und durch an einem Standort, beispielsweise einem Krankenhaus stehende Modalitäten erzeugte Bilder Bildbetrachtungs- und Bildbearbeitungsplätze, sogenannte Workstations, an einem in dem Standort installierten Bildkommunikationsnetz angeschlossen sind. Derartige Systemarchitekturen ermöglichen jedoch eine Kommunikation innerhalb des Standortes.

Die Erfindung geht von der Aufgabe aus, eine medizinische Systemarchitektur der eingangs genannten Art zu schaffen, die einen Abruf von Bildern oder Patientendaten auch von außerhalb beispielsweise eines Krankenhauses oder einer Arztpraxis ermöglicht, so daß ein Arzt Untersuchungen auf einfache Weise durchführen und so schnell wie möglich einen Befund eines anderen Arztes von einem entfernten Standort erhalten kann.

Die Aufgabe wird erfindungsgemäß dadurch gelöst, daß die medizinische Systemarchitektur mit einer Vorrichtung zur Erfassung von medizinischen Bilder und/oder Daten eine über ein Datennetz zur Übertragung der erfassten Bilder und/oder Daten mit der Vorrichtung verbundene, externe Speichervorrichtung zur Abspeicherung der erfassten Bilder und/oder Daten und eine über ein Datennetz mit der Speichervorrichtung verbundene Befundungsstation zur Auswertung der erfassten Bilder und/oder Daten, wobei die Daten der Auswertung in der Speichervorrichtung abgespeichert werden, so daß sie von einer Ausgabestation über ein Datennetz abrufbar sind. Dadurch wird erreicht, daß eine Befundung an einem anderen beliebigen Standort unabhängig von einem internen Datennetz erfolgen kann.

Es hat sich als vorteilhaft erwiesen, wenn die Ausgabestation eine an der Vorrichtung angeschlossene Sichtstation ist.

Ein einfacher, zentraler Aufbau wird erreicht, wenn ein Gateway an dem Datennetz angeschlossen ist, das eine Verteilung der Datenströme bewirkt.

Sowohl die Erfassungsstation als auch die Befundungsstation haben Zugriff zu verschiedenen Software-Funktionen und Bild- und Datenspeichern, wenn an dem Gateway mehrere Server mit unterschiedlichen Funktionen angeschlossen sind, die von jeder Befundungs- und/oder Sichtstation abrufbar sind.

In vorteilhafter Weise kann das Datennetz ein ISDN-Netz oder das Internet sein.

Es hat sich als vorteilhaft erwiesen, wenn die Vorrichtung zur Erfassung ein DICOM-Interface, ein Video-Interface, ein Internet-Interface oder ein Scanner-Interface aufweist.

Eine automatische Klassifikation von Wissen kann erfolgen, wenn an dem Gateway ein KOAN-Server zur Auswertung von Strukturen angeschlossen ist.

Die Aufgabe wird erfindungsgemäß auch durch ein Verfahren zur externen Befundung mit folgenden Schritten gelost:
a)Erfassung von medizinischen Bildern und/oder Daten,
b)Übertragung der erfassten Bilder und/oder Daten über ein Datennetz,
c)Speicherung der erfassten Bilder und/oder Daten in einer externen Speichervorrichtung,
d)Auswertung der gespeicherten Bilder und/oder Daten mit einer Befundungsstation,
e)Speicherung der ausgewerteten Daten in der Speichervorrichtung, und
f)Abrufung der Auswertung durch eine Ausgabestation.

Die Erfindung ist nachfolgend anhand eines in der Zeichnung dargestellten Ausführungsbeispieles näher erläutert. Es zeigen:
- Figur 1: eine Systemarchitektur eines medizinischen Bildkommunikationsnetzes und
- Figur 2: eine erfindungsgemäße Systemarchitektur.

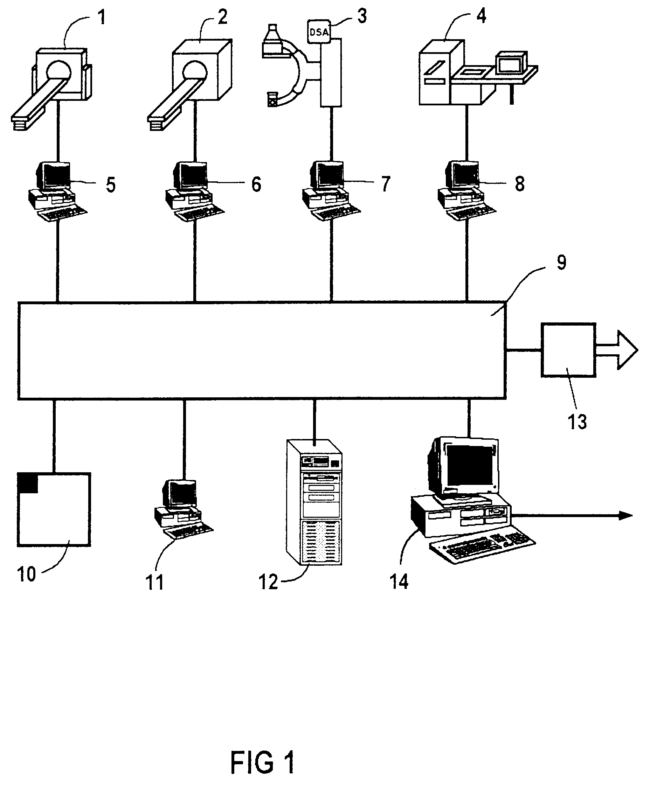

In der Figur 1 ist beispielhaft die Systemarchitektur eines medizinischen Bildkommunikationsnetzes in einem Fachzentrum oder Krankenhaus dargestellt. Zur Erfassung medizinischer Bilder dienen die Modalitäten 1 bis 4, die als bilderzeugende Systeme beispielsweise eine CT-Einheit 1 für Computertomographie, eine MR-Einheit 2 für Magnetische Resonanz, eine DSA-Einheit 3 für digitale Subtraktionsangiographie und eine Röntgeneinheit 4 für die digitale Radiographie 4 aufweisen kann. An diese Modalitäten 1 bis 4 können Workstations 5 bis 8 angeschlossen sein, mit denen die erfaßten medizinischen Bilder verarbeitet und lokal abgespeichert werden können. Auch lassen sich zu den Bildern gehörende Patientendaten eingeben. Eine derartige Workstation ist beispielsweise ein sehr schneller Kleincomputer auf der Basis eines oder mehrerer schneller Prozessoren.

Die Workstations 5 bis 8 sind mit einem Bildkommunikationsnetz 9 zur Verteilung der erzeugten Bilder und Kommunikation verbunden. So können beispielsweise die in den Modalitäten 1 bis 4 erzeugten Bilder und die in den Workstations 5 bis 8 weiter verarbeiteten Bilder in zentralen Bildspeicher- und Bildarchivierungssystemen 10 abgespeichert oder an andere Workstations weitergeleitet werden.

An dem Bildkommunikationsnetz 9 sind weitere Workstations 11 als Befundungskonsolen angeschlossen, die lokale Bildspeicher aufweisen. In den Workstations 11 können die erfaßten und im Bildspeichersystem 10 abgelegten Bilder nachträglich zur Befundung abgerufen und in dem lokalen Bildspeicher abgelegt werden, von dem sie unmittelbar der an der Workstation 11 arbeitenden Befundungsperson zur Verfügung stehen können.

Weiterhin sind an dem Bildkommunikationsnetz 9 Server 12, beispielsweise Patientendaten-Server (PDS), Fileserver und/oder Programm-Server, angeschlossen.

Der Bild- und Datenaustausch über das Bildkommunikationsnetz 9 kann dabei nach dem DICOM-Standard erfolgen, einem Industriestandard zur Übertragung von Bildern und weiteren medizinischen Informationen zwischen Computern zur Ermöglichung der digitalen Kommunikation zwischen Diagnose- und Theraphiegeräten unterschiedlicher Hersteller. An dem Bildkommunikationsnetz 9 kann ein Netzwerk-Interface 13 angeschlossen sein, über das das interne Bildkommunikationsnetz 9 mit einem globalen Datennetz verbunden ist, so daß die standardisierten Daten mit unterschiedlichen Netzwerken weltweit ausgetauscht werden können.

Es kann aber auch das Bildkommunikationsnetz 9 mit dem Datennetz des Krankenhausinformationssystem verbinden, so daß auch weitere Patientendaten abrufbar sind.

Weiterhin ist an dem internen Bildkommunikationsnetz 9 erfindungsgemäß ein PC oder eine weitere Workstation 14 angeschlossen, die ein Interface für ein ISDN-Netz oder das Internet aufweist, über das sie kommunizieren kann.

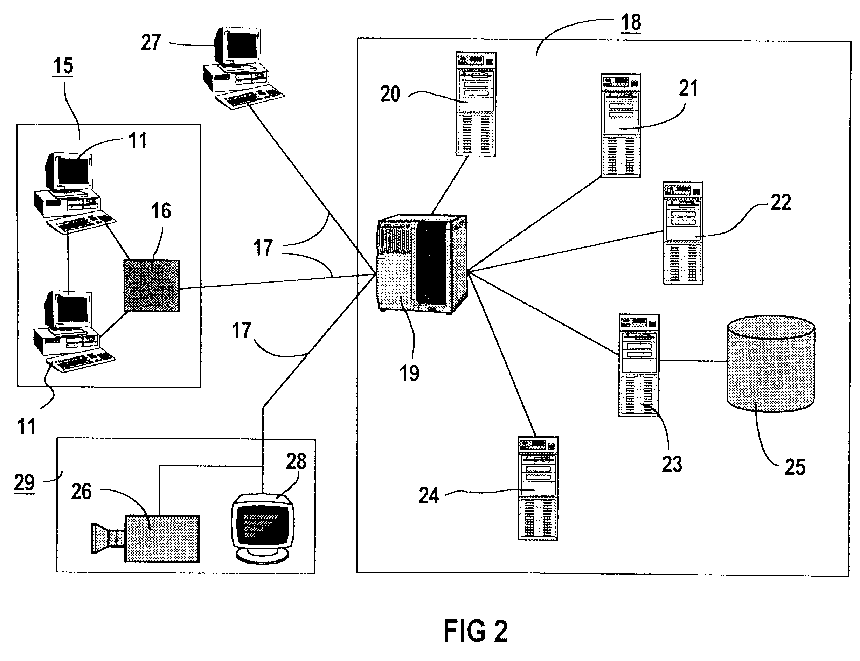

In der Figur 2 ist nun eine erfindungsgemäße Systemarchitektur dargestellt. In einem Krankenhaus 15 sind zwei symbolisch dargestellte Workstations 11 zur Patientendatenerfassung und Bildbefundung über das Bildkommunikationsnetz 9 miteinander verbunden. Weiterhin sind sie an einem ISDN-Interface 16 angeschlossen, das beispielsweise in der Workstation 14 angeordnet sein kann. Über das ISDN-Netz 17 ist das Krankenhaus 15 mit einem Provider 18 verbunden, bei dem ein Gateway 19 an dem ISDN-Netz 17 angeschlossen ist, das eine Verteilung der Datenströme bewirkt. An dem Gateway 19 kann ein Internet-Proxy-Server 20 zum Zugriff auf das Internet, ein sogenannter KOAN-Server 21 zur Auswertung von Strukturen in medizinischen Bildern, ein Knowledge Repository Server 22, ein Patientendaten-Server 23 zur Verwaltung der Patientendaten und Bilder und ein Kommunikations-Server 24 zur Zusammenarbeit aller Komponenten angeschlossen sein. Mit dem Patientendaten-Server 23 ist eine Objekt-orientierte Datenbank 25 als Speichervorrichtung verbunden.

Der KOAN-Server 21 kann dabei gemäß der aus der DE 44 38 589 C2 bekannten Lehre die Merkmalsstrukturen in medizinischen Bildern auswerten.

An dem Gateway 19 ist über das ISDN-Netz 17 als Beispiel eine Kamera 26 zur Erfassung von medizinischen Bildern angeschlossen, wie sie beispielsweise von einem in der deutschen Patentanmeldung 19650794.4 beschriebenen Gerät zur nichtinvasiven Messung von Vitalparametern verwendet wird. Weiterhin ist eine Befundungsstation 27 über das ISDN-Netz 17 mit dem Gateway 19 des Providers 18 verbunden.

Die Modalitäten 1 bis 4 können beispielsweise im Krankenhaus oder in Gemeinschaftspraxen von Ärzten angeordnet sein. Die Kamera 26 kann in einer Praxis 29 eines erstbehandelnden Arztes stehen. Die durch diese Modalitäten 1 bis 4 oder die Kamera 26 erstellten medizinischen Bilder werden erfindungsgemäß über ein Datennetz, dem ISDN-Netz 17, an den Provider 18 übertragen und dort mittels des Gateways 19 über den Patientendaten-Server 23 in der Datenbank 25 abgespeichert. Dort können sie von einem Facharzt über das ISDN-Netz 17 abgerufen werden und mittels der Befundungsstation 27 oder den Workstations 11 begutachtet werden. Der Facharzt übermittelt seinen Befund an den Provider 18, wo er in der Datenbank 25 abgespeichert wird. Der behandelnde Arzt kann ihn dort abrufen und als Ausgabestation mittels einer Sichtstation 28 oder Befundungsstation betrachten oder von einem Drucker ausdrucken lassen.

Eine externe Befundung wird also mit folgenden Schritten durchgeführt:
a)Erfassung von medizinischen Bildern und/oder Daten, beispielsweise durch die Modalitäten 1 bis 4 oder die Kamera 27,
b)Übertragung der erfassten Bilder und/oder Daten über ein Datennetz, beispielsweise das ISDN-Netz 17,
c)Speicherung der erfassten Bilder und/oder Daten in einer externen Speichervorrichtung, der Patientendatenbank 25,
d)Auswertung der gespeicherten Bilder und/oder Daten mit einer Befundungsstation 11 oder 27 durch einen Facharzt,
e)Speicherung der ausgewerteten Daten in der Patientendatenbank 25, und
f)Abrufung der Auswertung durch durch eine Ausgabestation, beispielsweise eine Sicht- oder Befundungsstation 11.

Somit können ein erster Befund oder nur die erfassten Bilder und Daten von einem Arzt in der Praxis oder in einem Krankenhaus einem Facharzt, der auch in einem Krankenhaus sitzen kann, ubermittelt werden, damit dieser Facharzt seinen Befund dazu abgeben kann. Dabei kann er die aus der US-Anmeldung 08/523,835 bekannte dynamische Bildbefundung anwenden.

Durch diese erfindungsgemäße medizinische Systemarchitektur sind neben der obengenannten Fernbefundung noch eine Beratung zwischen Arzt und Experten zur Erstellung eines zweiten Gutachtens, eine Online-Beratung eines Patienten durch den Arzt, eine Heimversorgung und Nachsorge sowie der Service und die Fernwartung denkbar.

Die erfindungsgemäße medizinische Systemarchitektur kann bestehende, führende Technologien wie Internet/Intranet, WWW Browser und Java verwenden. Diese Plattform ist offen für die Integration anderer Anwendungen.

Die erfindungsgemäßen, beim Provider vorgesehenen Server 20 bis 24 enthalten die objektorientierte Patientendatenbank 25, geben Zugang zum Internet und sammeln medizinische Informationen für eine spätere Auswertung (KOAN).

Die Hauptanwendung der Kunden ist ein Web-Browser beispielsweise Netscape Kommunikator oder Microsoft Internet Explorer. Die erfindungsgemäße Systemarchitektur verbessert die Basisleistungen des Browsers bei dem Zugang zu patientenorientierter medizinischer Information, Bildkommentaren für medizinische Multimedia-Berichte, 3D-Information-Visualisierung (KOAN), Videokonferenzen sowie gemeinsamen Zugriff auf Anwendungen und Audiokonferenzen.

Damit nur berechtigten Personen der Zugriff zu den Daten ermöglicht wird, kann ein auf Chipkarten basierendes Sicherheitssystem vorgesehen sein, das eine Verschlüsselungstechnologie nach strengen militärischen Standards verwendet.

## Patentansprüche

1. Medizinische Systemarchitektur mit einer Vorrichtung (1 bis 4, 26) zur Erfassung von medizinischen Bilder und/oder Daten, mit einer über ein Datennetz (17) zur Übertragung der erfassten Bilder und/oder Daten mit der Vorrichtung (1 bis 4, 26) verbundene, externe Speichervorrichtung (25) zur Abspeicherung der erfassten Bilder und/oder Daten, mit einer über ein Datennetz (17) mit der Speichervorrichtung (25) verbundenen Befundungsstation (27) zur Auswertung der erfassten Bilder und/oder Daten, wobei die Daten der Auswertung in der Speichervorrichtung (25) abgespeichert werden, so daß sie von einer Ausgabestation (28) über ein Datennetz (17) abrufbar sind.

2. Medizinische Systemarchitektur nach Anspruch 1, **dadurch gekennzeichnet,** daß die Ausgabestation eine an der Vorrichtung angeschlossene Sichtstation (28) ist.

3. Medizinische Systemarchitektur nach Anspruch 1 oder 2, **dadurch gekennzeichnet,** daß ein Gateway (19) an dem Datennetz (17) angeschlossen ist, das eine Verteilung der Datenströme bewirkt.

4. Medizinische Systemarchitektur nach Anspruch 1 oder 2, **dadurch gekennzeichnet,** daß an dem Gateway (19) mehrere Server (20 bis 24) mit unterschiedlichen Funktionen angeschlossen sind, die von jeder Befundungs- (27) und/oder Sichtstation (28) abrufbar sind.

5. Medizinische Systemarchitektur nach einem der Ansprüche 1 bis 3, **dadurch gekennzeichnet,** daß das Datennetz ein ISDN-Netz (17) ist.

6. Medizinische Systemarchitektur nach einem der Ansprüche 1 bis 3, **dadurch gekennzeichnet,** daß das Datennetz (17) das Internet ist.

7. Medizinische Systemarchitektur nach einem der Ansprüche 1 bis 6, **dadurch gekennzeichnet,** daß die Vorrichtung zur Erfassung ein DICOM-Interface aufweist.

8. Medizinische Systemarchitektur nach einem der Ansprüche 1 bis 6, **dadurch gekennzeichnet,** daß die Vorrichtung zur Erfassung ein Video-Interface aufweist.

9. Medizinische Systemarchitektur nach einem der Ansprüche 1 bis 6, **dadurch gekennzeichnet,** daß die Vorrichtung zur Erfassung ein Internet-Interface aufweist.

10. Medizinische Systemarchitektur nach einem der Ansprüche 1 bis 6, **dadurch gekennzeichnet,** daß die Vorrichtung zur Erfassung ein Scanner-Interface aufweist.

11. Medizinische Systemarchitektur nach einem der Ansprüche 4 bis 10, **dadurch gekennzeichnet,** daß an dem Gateway (19) ein KOAN-Server (21) zur Auswertung von Strukturen angeschlossen ist.

12. Verfahren zur externen Befundung **gekennzeichnet durch** folgende Schritte:
a)Erfassung von medizinischen Bildern und/oder Daten,
b)Übertragung der erfassten Bilder und/oder Daten über ein Datennetz (17),
c)Speicherung der erfassten Bilder und/oder Daten in einer externen Speichervorrichtung (25),
d)Auswertung der gespeicherten Bilder und/oder Daten mit einer Befundungsstation (27),
e)Speicherung der ausgewerteten Daten in der Speichervorrichtung (25), und
f)Abrufung der Auswertung durch eine Ausgabestation (28).
